# EUROPEAN PATENT SPECIFICATION

(11) **EP 3 427 783 B1**
(45) Date of publication and mention of the grant of the patent: **04.12.2019**
(21) Application number: 17763507.5
(22) Date of filing: 03.03.2017
(51) Int. Cl.: A61M 16/04, A61B 1/05, A61B 1/267, A61B 1/04, A61B 1/00

(54) **DISPOSABLE INTUBATION APPARATUS**
EINWEG-INTUBATIONSVORRICHTUNG
APPAREIL D'INTUBATION JETABLE

(30) Priority: 08.03.2016 KR 20160027442
(43) Date of publication of application: 16.01.2019
(73) Proprietor: Medicalpark Co., Ltd., Yongin-si, Gyeonggi-do 16827 (KR)
(72) Inventor: PARK, Hee Boong, Seoul 06281 (KR)
(74) Representative: v. Bezold & Partner Patentanwälte - PartG mbB
(86) International application number: PCT/KR2017/002316
(87) International publication number: WO 2017/155245

(56) References cited:
- EP-A1- 2 902 066
- EP-A2- 2 567 725
- WO-A2-2015/013172
- JP-A- 2014 036 868
- KR-A- 20140 004 560
- KR-B1- 100 894 709
- KR-B1- 101 377 936
- US-A1- 2007 215 162
- US-A1- 2011 270 034
- US-A1- 2014 275 772

## Description

### Technical Field

The present invention relates to an intubation apparatus and, more specifically, to a disposable intubation apparatus which can be easily intubated to perform artificial respiration on a patient.

### Background Art

Intubation is performed to perform artificial respiration on a patient who is undergoing general anesthesia for an operation, a patient who is in an unconscious state due to an accident, or a critical patient who is difficult to self-breathe.

Conventionally, a laryngoscope is used to perform intubation for artificial respiration. In order to insert a tube into a patient's organ, the patient is laid down and the head is tilted backward. Then, the laryngoscope is inserted into the oral cavity so that the operator can check the vocal cords of the larynx and can insert a tube through between the vocal cords. However, it is very difficult to use the laryngoscope when the patient is conscious, and it may be dangerous to tilt the head backward when the patient has a problem with his or her neck. It is also difficult to use a laryngoscope for intubation on a patient with a small oral cavity and jaw or a patient with a short neck. In addition, when the laryngoscope is used, the teeth may be broken in a person whose teeth are not strong.

Instead of the laryngoscope, an endoscope may be used to perform intubation. However, the endoscope is expensive. In order to use the endoscope, the user need special dexterity and must learn professional usage.

In order to insert a tube into the oral cavity of a patient using a laryngoscope, the operator holds the laryngoscope with one hand and performs the intubation with the other hand. If hemorrhage occurs in the oral cavity of the patient or if the secretion in the oral cavity of the patient is excessive, aspiration should be performed to remove foreign substances existing in the oral cavity of the patient. In this case, the operator cannot independently perform the intubation while removing the foreign substances. Even if intubation is performed using an endoscope, the operator cannot independently perform the intubation while removing the foreign substances existing in the oral cavity.

In addition, the laryngoscope or the endoscope used once must be disinfected in order to use the same with other patients. It takes a lot of time and money to disinfect the laryngoscope or the endoscope. If the disinfection is insufficient, the patent may be infected with another patient's disease.

US 2014/275772 A1 discloses an intubation device with video and anatomic stylet steering. EP 2567725 A2 discloses tracheal intubation device. US 2007/215162 A1 discloses visualization airway apparatus. US 2011/0270034 A1 discloses endotracheal tube with side mounted camera. But all the inventions mentioned above do not disclose a disposable intubation device that have simple structure so that it can be made in low cost.

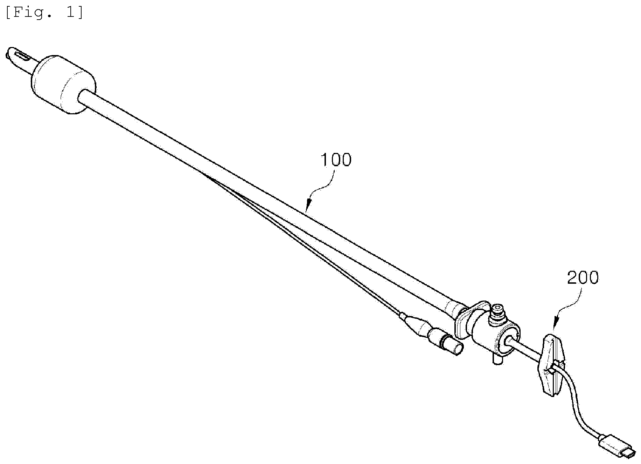

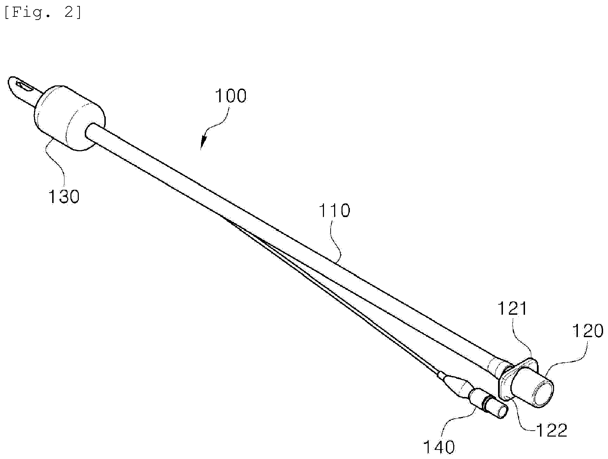

EP 2 902 066 A1 discloses an airway cleaning and visualization device according to the preamble of claim 1. Referring to Fig 1 and Fig 2 of the patent specification, it include a hollow guide tube (102) and a visualization member (103). The visualization member (103) can comprise an imaging element and light delivery elements. The imaging element can be a camera and the light delivery elements can be optical fibers. Therefore the device is expensive and cannot be a disposable one. Further, the mounting of the camera within the guide tube is not entirely satisfactory.

Therefore, it is an object of the invention to provide an improved disposable intubation apparatus.

The invention is defined by the appended claims.

### Summary of the Invention

The present invention is intended to solve the above-mentioned problems that arise in the case of performing intubation through the use of a laryngoscope or an endoscope.

It is an object of the present invention to provide an intubation apparatus that allows an operator alone to easily perform intubation. Another object of the present invention is to provide a low-cost disposable intubation apparatus capable of preventing a patient from being infected by intubation when the intubation is performed using a laryngoscope.

The disposable intubation apparatus according to the present invention includes an intubation tube and a hollow guide tube which is inserted into a bore of the intubation tube to facilitate insertion of the intubation tube. The intubation tube is a hollow tube having opposite open ends, in which one end thereof is fixed to an adapter to be connected to an external device for supplying a gas to the bore. The guide tube has open first and second ends and is arranged to be inserted into the intubation tube so that the second end is exposed to the outside of the adapter. The disposable intubation apparatus according to the present invention includes a receptacle which is detachably connected to the adapter of the intubation tube so as to facilitate insertion of the intubation tube using the guide tube. The receptacle is formed in a hollow cylindrical shape. The receptacle has one open end detachably coupled to the adapter. The receptacle is disposed so that the second end of the guide tube can be exposed through the other end of the receptacle.

In addition, the disposable intubation apparatus according to the present invention includes a lens installed at the first end of the guide tube such that an operator can identify vocal cords and bronchial tubes when inserting the guide tube into the oral cavity, and a camera module installed behind the lens. An LED lamp is included in the camera module. The camera module is connected to a USB cable, and a USB cable terminal is arranged to be exposed toward the second end of the guide tube. The lens is installed at the terminal end of the first end of the guide tube so as to seal the bore of the guide tube.

In the disposable intubation apparatus according to the present invention, the guide tube is made of a material having such elasto-plasticity that the guide tube can maintain a bent shape when the first end of the guide tube is bent at a predetermined angle or more. For example, an aluminum alloy pipe having an appropriate diameter and an appropriate thickness, or a synthetic resin pipe including a steel wire having an appropriate diameter may be used as the guide tube. Prior to insertion of the intubation tube, depending on the condition of a patient, the first end of the guide tube is bent at an appropriate angle. For example, the guide tube may be made so as to have elasto-plasticity in the range of 30 to 90 degrees.

In some embodiments, the disposable intubation apparatus according to the present invention may further include a handle fixed to the second end of the guide tube so as to easily remove the guide tube after intubation.

In some embodiments, the receptacle may include a suction nipple protruding from an outer circumferential surface of the receptacle so as to communicate with a bore of the receptacle in order to remove foreign substances existing in the oral cavity of a patient.

In some embodiments, When the USB terminal of the disposable intubation apparatus is connected to a smart phone, a smart pad, a portable computer or the like, an image in the oral cavity may be displayed on a display of a device such as a smart phone or the like. Thus, an ordinary person who receives a simple education can secure the airway of an emergency patient by safely and promptly performing intubation on the emergency patient while confirming an image displayed on the display.

The disposable intubation apparatus according to the present invention can promptly and safely perform intubation on a patient while confirming an image of the interior of the oral cavity displayed on a display by using a guide tube provided with a camera at a distal end. Further, the intubation apparatus according to the present invention includes a guide tube and does not need to use a laryngoscope. Since it is not necessary to disinfect and reuse a laryngoscope, there is no risk of infection due to poor laryngoscope disinfection. Inasmuch as the intubation apparatus according to the present invention is disposable, it is convenient to use because of no need of disinfection for reuse and it is hygienic because of no risk of infection.

### Brief Description of the Drawings

FIG. 1 is a perspective view of a disposable intubation apparatus according to an embodiment of the present invention.
FIG. 2 is a perspective view of an intubation tube.
FIG. 3 is a perspective view of a guide tube assembly.
FIG. 4 is an enlarged sectional view of the guide tube assembly.
FIG. 5 is a sectional view of a receptacle.

### Detailed Description

Hereinafter, a preferred embodiment of the present invention will be described in detail with reference to the accompanying drawings.

Referring to FIG. 1, the disposable intubation apparatus of the present embodiment includes an intubation tube 100 and a guide tube assembly 200 inserted into a bore of the intubation tube 100 to facilitate intubation of the intubation tube 100.

Referring to FIG. 2, the intubation tube 100 includes a hollow ventilation tube 110 having opposite open ends, and an adapter 120 fixed to one end of the ventilation tube 110 and connected to an external device for supplying a gas into the ventilation tube 110. The ventilation tube 110 is made of a flexible semi-rigid plastic such as polyethylene, polypropylene or the like. A balloon 130 is formed at the other end of the ventilation tube 110, and a socket 140 for injecting an air into the balloon 130 is connected to the balloon 130. A syringe is connected to the socket 140 to supply an air to the balloon 130. After intubation is completed, the balloon 130 is inflated to prevent the air supplied to the lung from escaping to the outside during artificial respiration. After the intubation is performed, the adapter 120 is connected to a device such as a respirator or the like to supply an air to the ventilation tube 110. The adapter 120 includes a connecting portion 121 having a larger diameter than the ventilation tube 110 and a collar 122 for defining a boundary of the connecting portion 121.

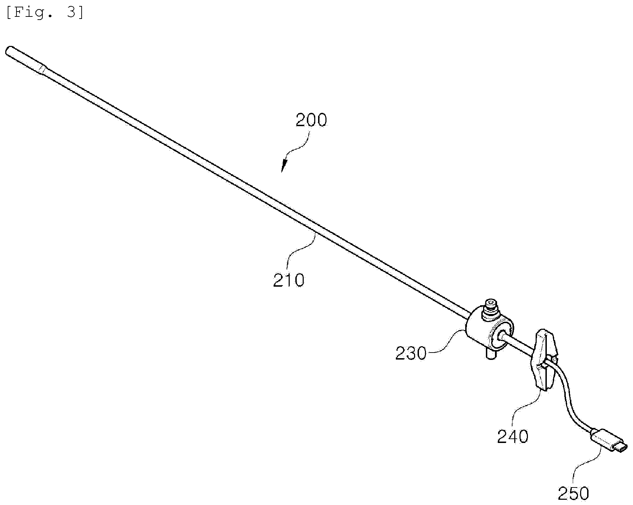

Referring to FIG. 3, the guide tube assembly 200 includes a guide tube 210, a receptacle 230 and a handle 240. The guide tube 210 has an open first end and an open second end. The guide tube 210 is inserted into the ventilation tube 110 of the intubation tube 100 and is disposed so that the second end is exposed to the outside of the adapter 120. The guide tube 210 is larger in length and smaller in diameter than the intubation tube 100. The guide tube 210 is inserted into the bore of the intubation tube 110 to facilitate insertion of the intubation tube 100.

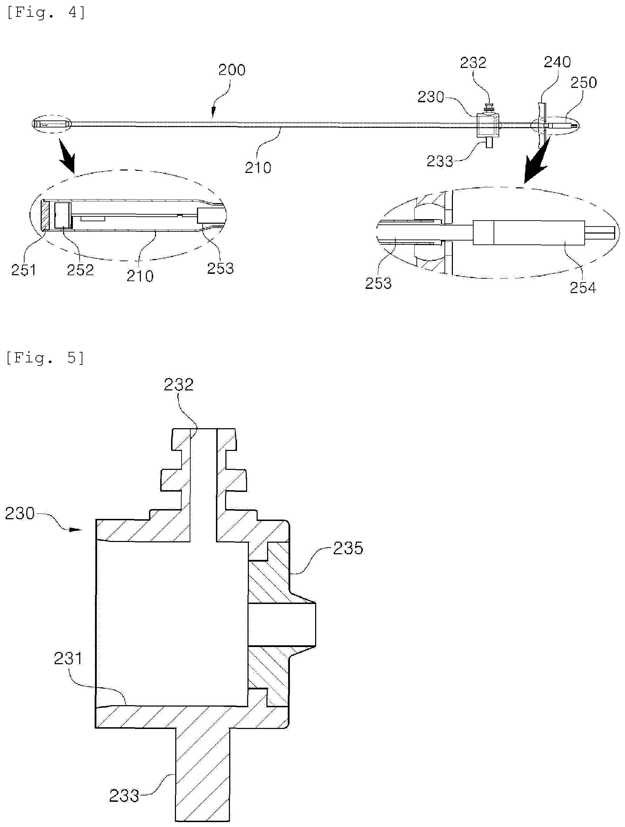

Referring to FIGS. 1 and 5, the receptacle 230 has a hollow cylindrical shape. The receptacle 230 is opened at one end and is detachably coupled to the adapter 120. A cap 235 is fixed to the other end of the receptacle 230 and is disposed so that the second end of the guide tube 210 passes through the center of the cap 235 and is exposed to the outside. The receptacle 230 is detachably connected to the adapter 120 of the intubation tube 100 and is detached and removed from the intubation tube 100 together with the guide tube 210 when the insertion of the intubation tube 100 is completed. When the receptacle 230 and the guide tube 210 are removed from the intubation tube 100 after the intubation is completed, a respirator is connected to the adapter 120. A suction nipple 232 protruding so as to communicate with the hollow interior 231 is formed on the outer circumferential surface of the receptacle 230. A vacuum pipe is connected to the suction nipple 232 so that the suction nipple 232 can be used to remove foreign substances existing in the oral cavity of a patient. In addition, a handle 233 for holding the receptacle 232 by the user when the vacuum pipe is connected to the receptacle 232 is formed on the outer circumferential surface of the receptacle 230.

The guide tube 210 is preferably made of a material having such elasto-plasticity that the guide tube 210 can maintain a bent shape when the first end portion thereof is bent at a predetermined angle or more. For example, an aluminum alloy pipe having an appropriate diameter and an appropriate thickness, or a synthetic resin pipe including a steel wire having an appropriate diameter may be used as the guide tube 210. For example, the guide tube 210 may be made so as to have elasto-plasticity in the range of 30 to 90 degrees. Prior to insertion of the intubation tube 100 into the oral cavity of a patient, depending on the condition of the patient, the end portion of the guide tube 210 may be bent at an appropriate angle to facilitate insertion into the bronchus.

The handle 240 is fixed to the second end of the guide tube 210 so that the guide tube 210 and the receptacle 230 can be easily removed by pulling the handle 240 after the insertion of the intubating tube 100 is completed.

Referring to FIG. 4, the disposable intubation apparatus includes an image module 250 for acquiring an image so that an operator can identify the vocal cords and the bronchial tubes when the guide tube 210 is inserted into the oral cavity. The image module 250 includes a lens 251, a camera module 252, a USB cable 253 and a USB cable terminal 254. The lens 252 is installed at one end of the guide tube 210 to seal the guide tube 210, thereby preventing foreign substances from entering into the bore of the guide tube 210. The camera module 252 is provided in the bore of the guide tube 210 on the rear side of the lens 251. An LED lamp (not shown) is usually included in the camera module 252. The camera module 252 is connected to the USB cable 253, and the USB cable terminal 254 is disposed so as to be exposed to the second end of the guide tube 210. According to the invention, first end of the guide tube 210 is expanded, and the camera module is inserted into the expanded portion. By installing the camera module 252 in the expanded portion, the camera module 252 can be prevented from moving in the guide tube 210 and can be securely mounted.

When the USB terminal 254 of the disposable intubation apparatus is connected to a smartphone, the APP of the smartphone is operated to turn on the LED of the camera module so that the image of the oral cavity can be displayed on the display of the smartphone. Thus, according to the intubation apparatus, an ordinary person who receives a simple education can secure the airway of an emergency patient by safely and promptly performing intubation on the emergency patient while confirming an image displayed on the display.

## Claims

1. A disposable intubation apparatus, comprising:
a) an intubation tube (110) formed in a hollow tube shape so as to have opposite open ends, the intubation tube (110) including an adapter (120) fixed to one end of the intubation tube (110) so that the adapter (120) can be connected to an external device for supplying a gas to a bore of the intubation tube (110);
b) a hollow guide tube (210) having an open first end and an open second end, the guide tube (210) inserted into the intubation tube (110) and disposed so that the second end thereof is exposed to the outside of the adapter (120), the guide tube (210) being larger in length and smaller in diameter than the intubation tube (110);
c) a receptacle (230) formed in a hollow cylindrical shape, the receptacle (230) having one open end detachably coupled to the adapter (120), the receptacle (230) disposed so that the second end of the guide tube (210) can be exposed through the other end of the receptacle (230) ;
d) a lens (251) provided at the first end of the guide tube (210) to seal a bore of the guide tube (210);
e) a camera module (252) installed inside the guide tube (210) on a rear side of the lens (251); and
f) a USB cable (253) electrically connected at one end to the camera module (252) and disposed so as to be exposed to the second end of the guide tube (210) through the bore of the guide tube (210),
g) wherein the guide tube (210) is made of a material having such elasto-plasticity that the guide tube (210) can maintain a bent shape when the first end of the guide tube (210) is bent at a predetermined angle or more,
**characterized in that**
h) the first end of the guide tube (210) is provided with an expanded portion, and
i) the camera module (252) is inserted into the expanded portion, so that the camera module (252) can be prevented from moving in the guide tube (210) and can be securely mounted.

2. The apparatus of claim 1, further comprising:
a handle (240) fixed to the second end of the guide tube (210) .

3. The apparatus of claim 1, wherein the receptacle includes a suction nipple (232) protruding from an outer circumferential surface of the receptacle (230) so as to communicate with a bore of the receptacle (230).

4. The apparatus of claim 1, wherein the guide tube (210) is made of an aluminum alloy.

## Patentansprüche

1. Wegwerfbare Intubationsvorrichtung, umfassend:
a) ein Intubationsrohr (110), das in einer hohlen Rohrform ausgebildet ist, um gegenüberliegende offene Enden zu haben, wobei das Intubationsrohr (110) einen Adapter (120) beinhaltet, der an einem Ende des Intubationsrohrs (110) befestigt ist, so dass der Adapter (120) mit einer externen Vorrichtung zum Zuführen eines Gases zu einer Bohrung des Intubationsrohrs (110) verbunden werden kann;
b) ein hohles Führungsrohr (210) mit einem offenen ersten Ende und einem offenen zweiten Ende, wobei das Führungsrohr (210) in das Intubationsrohr (110) eingesetzt und so angeordnet ist, dass sein zweites Ende der Außenseite des Adapters (120) ausgesetzt ist, wobei das Führungsrohr (210) größer in der Länge und kleiner im Durchmesser als das Intubationsrohr (110) ist;
c) eine Aufnahme (230), die in einer hohlzylindrischen Form ausgebildet ist, wobei die Aufnahme (230) ein offenes Ende aufweist, das lösbar mit dem Adapter (120) gekoppelt ist, wobei die Aufnahme (230) so angeordnet ist, dass das zweite Ende des Führungsrohrs (210) durch das andere Ende der Aufnahme (230) freigelegt werden kann;
d) eine Linse (251), die am ersten Ende des Führungsrohrs (210) vorgesehen ist, um eine Bohrung des Führungsrohrs (210) abzudichten;
e) ein Kameramodul (252), das innerhalb des Führungsrohrs (210) auf einer Rückseite der Linse (251) installiert ist; und
f) ein USB-Kabel (253), das an einem Ende elektrisch mit dem Kameramodul (252) verbunden und so angeordnet ist, dass es dem zweiten Ende des Führungsrohrs (210) durch die Bohrung des Führungsrohrs (210) ausgesetzt ist,
g) wobei das Führungsrohr (210) aus einem Material mit einer solchen Elastoplastizität hergestellt ist, dass das Führungsrohr (210) eine gebogene Form beibehalten kann, wenn das erste Ende des Führungsrohrs (210) in einem vorbestimmten Winkel oder mehr gebogen wird,
**dadurch gekennzeichnet, dass**
h) das erste Ende des Führungsrohrs (210) mit einem erweiterten Abschnitt versehen ist, und
i) das Kameramodul (252) in den erweiterten Abschnitt eingesetzt ist, so dass das Kameramodul (252) an der Bewegung im Führungsrohr (210) gehindert werden kann und sicher montiert werden kann.

2. Vorrichtung nach Anspruch 1, ferner umfassend:
einen Griff (240), der am zweiten Ende des Führungsrohrs (210) befestigt ist.

3. Vorrichtung nach Anspruch 1, wobei der Behälter einen Saugnippel (232) beinhaltet, der von einer äußeren Umfangsfläche des Behälters (230) vorsteht, um mit einer Bohrung des Behälters (230) zu kommunizieren.

4. Vorrichtung nach Anspruch 1, wobei das Führungsrohr (210) aus einer Aluminiumlegierung hergestellt ist.

## Revendications

1. Appareil d'intubation jetable, comprenant:
a) un tube d'intubation (110) formé en forme de tube creux de manière à avoir des extrémités ouvertes opposées, le tube d'intubation (110) comprenant un adaptateur (120) fixé à une extrémité du tube d'intubation (110) de sorte que l'adaptateur (120) peut être connecté à un dispositif externe pour fournir un gaz à un orifice du tube d'intubation (110);
b) un tube de guidage creux (210) ayant une première extrémité ouverte et une seconde extrémité ouverte, le tube de guidage (210) étant inséré dans le tube d'intubation (110) et disposé de telle sorte que sa seconde extrémité soit exposée à l'extérieur de l'adaptateur (120), le tube de guidage (210) ayant une longueur plus grand et un diamètre inférieur que le tube d'intubation (110);
c) un réceptacle (230) de forme cylindrique creuse, le réceptacle (230) ayant une extrémité ouverte couplée de manière amovible à l'adaptateur (120), le réceptacle (230) étant disposé de telle sorte que la deuxième extrémité du tube de guidage (210) puisse être exposée par l'autre extrémité du réceptacle (230);
d) une lentille (251) prévue à la première extrémité du tube de guidage (210) pour obturer un alésage du tube de guidage (210);
e) un module caméra (252) installé à l'intérieur du tube de guidage (210) sur un côté arrière de la lentille (251); et
f) un câble USB (253) connecté électriquement à une extrémité au module de caméra (252) et disposé de manière à être exposé à la deuxième extrémité du tube de guidage (210) par l'alésage du tube de guidage (210),
g) dans lequel le tube de guidage (210) est fait d'un matériau ayant une élasticité telle que le tube de guidage (210) peut maintenir une forme courbée lorsque la première extrémité du tube de guidage (210) est courbée à un angle prédéterminé ou plus,
**caractérisé en ce que**
h) la première extrémité du tube de guidage (210) est munie d'une partie dilatée, et
i) le module de caméra (252) est inséré dans la partie étendue, de sorte que le module de caméra (252) peut être empêché de se déplacer dans le tube de guidage (210) et peut être monté de manière sûre.

2. Appareil selon la revendication 1, comprenant en outre:
une poignée (240) fixée à la deuxième extrémité du tube de guidage (210).

3. Appareil selon la revendication 1, dans lequel le récipient comprend un raccord d'aspiration (232) dépassant d'une surface circonférentielle extérieure du récipient (230) de manière à communiquer avec un trou du récipient (230).

4. Appareil selon la revendication 1, dans lequel le tube de guidage (210) est réalisé en un alliage d'aluminium.
